# EUROPEAN PATENT APPLICATION

(11) **EP 1 550 448 A1**
(43) Date of publication of application: **06.07.2005**
(21) Application number: 03797700.6
(22) Date of filing: 19.09.2003
(51) Int. Cl.: A61K 31/7016, A61K 31/702, A61K 31/732, A61K 7/00, A61P 1/04, A61P 11/02, A61P 11/06, A61P 17/00, A61P 17/04, A61P 27/14, A61P 43/00, C08B 37/06, C07H 3/04, C07H 3/06

(54) **HISTAMINE RELEASE INHIBITOR**

(30) Priority: 20.09.2002 JP 2002275368
(71) Applicant: Incorporated Administrative Agency, National Agriculture and Bio-Oriented Research Organization, Tsukuba-shi, Ibaraki 305-8517 (JP)
(72) Inventor: TANAKA, Keiichi, Tsuchiura-shi, Ibaraki 300-0844 (JP); AMANO, Takayuki, Tsukuba-shi, Ibaraki 305-0046 (JP); MURAMATSU, Noboru, Zentsuji-shi, Kagawa 765-0004 (JP); TATSUKI, Miho, Tsukuba-shi, Ibaraki 305-0044 (JP); ASAKURA, Toshikazu, Tsuchiura-shi, Ibaraki 300-0815 (JP); ITO, Iwao, Tsuchiura-shi, Ibaraki 300-0022 (JP); ISHIKAWA, Etsuo, Tsukuba-shi, Ibaraki 305-0035 (JP); SATO, Koichi, Tsukuba-shi, Ibaraki 305-0834 (JP)
(74) Representative: Woods, Geoffrey Corlett
(86) International application number: PCT/JP2003/012002
(87) International publication number: WO 2004/026317

(57) **Abstract**

The present invention provides a histamine release inhibitor comprising a pectin or a salt thereof or a pectin hydrolysate as an active ingredient, and a pharmaceutical composition, a cosmetic, and food and drink comprising the inhibitor.

## Description

### TECHNICAL FIELD

The present invention relates to a histamine release inhibitor capable of preventing and/or treating diseases such as allergic rhinitis, atopic dermatitis, urticaria, bronchial asthma, peptic ulcer, and pollinosis, and to a pharmaceutical composition, a cosmetic, and food and drink comprising the inhibitor.

### BACKGROUND ART

Histamine plays an important role in inflammation and immediate allergic diseases. Immunological stimulation induces degranulation of mast cells and basocytes, during which *in vivo* chemotransmitters such as histamine, leukotrien, and serotonin are released into the blood.

Among the above *in vivo* chemotransmitters, histamine in particular has been the most studied. Histamine released in blood binds to the histamine receptors of various organs, inducing symptoms such as increased blood vessel permeability, smooth muscle atrophy, and increased mucous secretion. As a result, it is known that diseases such as allergic rhinitis, atopic dermatitis, urticaria, bronchial asthma, peptic ulcer, and pollinosis may be induced (see e.g., White, M. V. et al., "Journal of Allergy and Clinical Immunology," 1990, 86: pp. 599-605).

Furthermore, it has been reported that when bronchoalveolar lavage is performed for a bronchial asthma patient, the resulting histamine concentration in a washing solution becomes significantly higher than that of a normal healthy person (see e.g., Casale, T. B. et al., "Journal of Clinical Investigation," 1987, 79: pp. 1197-1203).

Hence, to inhibit allergies and inflammation, it is necessary to inhibit *in vivo* chemotransmitters such as histamine from being released into the blood.

Currently, the two following methods are employed for treating allergic diseases caused by histamine. A 1st method is to block histamine receptors by administering a chemically synthesized product referred to as an antihistamic agent. A 2nd method is to inhibit histamine release from mast cells by administering a mast cell histamine release inhibitor.

However, taking an antihistamine agent according to the former method is problematic in that sleepiness, vertigo, or dullness may be caused or in that a side effect such as arrhythmia may be caused. By taking such an antihistamine agent, diseases can be temporarily treated, but continuous taking of such an agent for the purpose of prevention is dangerous. In the case of the latter method, experiments at the cellular level have been conducted using a histamine release inhibitor in mast cells. However, when the mast cell histamine inhibitor is actually taken or applied, it is problematic in that it is unclear whether or not it is transferred in the blood and directly acts on mast cells.

Therefore, histamine release inhibitors that are safe and have been confirmed to have a definite effect when they are taken or applied are desired for preventing and/or treating allergic diseases.

In the meantime, the association of diet with the incidence of chronic nonspecific lung diseases such as asthma, bronchitis, and pulmonary emphysema has been investigated for 25 years (1960-1985) in a study of the inhabitants of the area of Zutphen, in the Netherlands (see Miedema, I. et al., "American Journal of Epidemiology," 1993, 138: pp. 37-45). As a result, no significant differences have been observed between the intake of vegetables and fish and the onset of the above chronic nonspecific lung diseases. However, the intake of fruits inhibited the onset of these diseases, confirming a negative correlation between the intake of fruits and the above chronic nonspecific lung diseases (relative risk of 0.73: 95% confidence interval ranging from 0.53 to 0.99). In particular, when persons taking 70 g or more of apple or pear per day were compared with persons taking 14 g or less of the same, the relative risk was found to be 0.63 (95% confidence interval ranging from 0.45 to 0.88). Accordingly, the risk of chronic nonspecific lung diseases such as asthma can be reduced by 37% by the intake of apples or pears. This has revealed that chronic nonspecific lung diseases such as bronchial asthma can be prevented by the daily intake of large quantities of fruit (particularly apple and pear). However, this report does not disclose about which ingredients contained in fruits, including apples and pears, are effective in the prevention of chronic nonspecific lung diseases such as bronchial asthma.

Furthermore, Yamada et al., conducted an animal experiment using rats wherein the dietary effects obtained by the intake of water-soluble dietary fibers such as guar gum, glucomannan, and high methoxyl pectin on serum lipid concentration and IgA production were examined and compared with those of insoluble cellulose. It was reported that serum IgA concentration was increased by the intake of water-soluble dietary fibers (see Yamada, K. et al., "Bioscience, Biotechnology, and Biochemistry," 1999, 63: pp. 2163-2167). However, this report does not describe the relationship between pectin and IgE antibody production involved in allergic reaction and does not disclose any effect of pectin on histamine production at all.

### SUMMARY OF THE INVENTION

An object of the present invention is to provide: a histamine release inhibitor that is safe if it is taken daily and is capable of preventing and/or treating diseases such as allergic rhinitis, atopic dermatitis, urticaria, bronchial asthma, peptic ulcer, and pollinosis; and a pharmaceutical composition, a cosmetic, and food and drink comprising the inhibitor.

As a result of intensive studies to achieve the above object, we have found that pectin that is already widely used as a food material has action to lower histamine concentration in blood, and thus we have completed the present invention.

The present invention provides the following (1) to (9).
(1) A histamine release inhibitor, comprising a pectin or a salt thereof or a pectin hydrolysate as an active ingredient.
(2) The histamine release inhibitor of (1), wherein the pectin is a high methoxyl pectin having an esterification degree of 50% or more and 90% or less.
(3) The histamine release inhibitor of (1), wherein the pectin is a low methoxyl pectin having an esterification degree of 3% or more and less than 50%.
(4) The histamine release inhibitor of (1), wherein the pectin hydrolysate is oligogalacturonic acid and/or polygalacturonic acid with a polymerization degree between 2 and 100.
(5) The histamine release inhibitor of (1), wherein the pectin hydrolysate is an oligosaccharide comprising 1 type or 2 or more types selected from the group consisting of galacturonic acid, rhamnose, arabinose, xylose, fucose, and galactose as a constituent.
(6) The histamine release inhibitor of any one of (1) to (5), which is for preventing and/or treating allergic rhinitis, atopic dermatitis, urticaria, bronchial asthma, peptic ulcer, and/or pollinosis.
(7) A pharmaceutical composition, comprising the inhibitor of any one of (1) to (6).
(8) A cosmetic, comprising the inhibitor of any one of (1) to (6).
(9) Food and drink, comprising the inhibitor of any one of (1) to (6).

This specification includes part or all of the contents as disclosed in the specification and/or drawings of Japanese Patent Application No. 2002-275368, which is a priority document of the present application.

### BRIEF DESCRIPTION OF THE DRAWINGS

Fig. 1 shows an example of the structure of an apple pectin.
Fig. 2 shows periods of pectin intake and timing of blood collection.
Fig. 3 shows fluctuations in histamine concentration in the blood of each subject during an experiment period.

### BEST MODE OF CARRYING OUT THE INVENTION

The present invention will be further described in detail as follows.

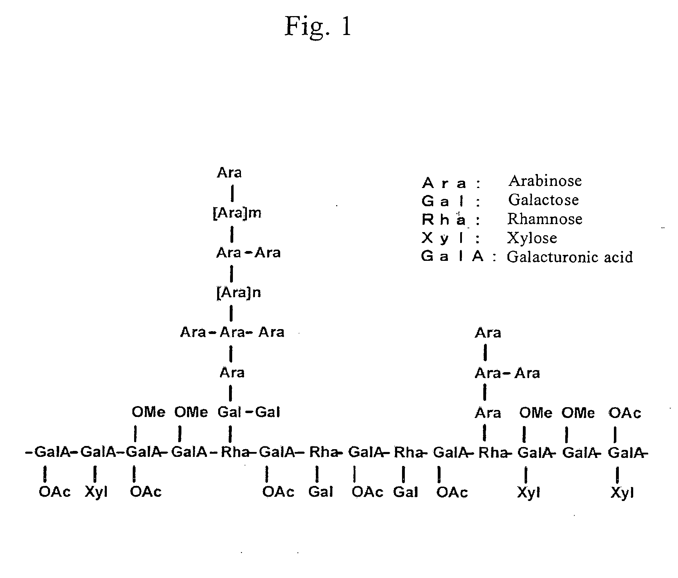

The pectin having action to inhibit histamine release of the present invention is also referred to as a pectic substance that is a constituent of a plant cell wall and is an acidic polysaccharide broadly existing in nature. The chemical structure of pectin (pectic substance) is composed of the main chain (rhamnogalacturonan) wherein: D-galacturonic acids are linked by α-1,4 bonds to form polygalacturonic acid (polygalacturonan) and L-rhamnose that is linked by α-1,2 bonds to a part of the polygalacturonic acid and thus coexists therewith; and side chains composed of neutral sugars such as arabinose, xylose, fucose, rhamnose, and galactose linked to the main chain, thereby forming a pectinate structure. The side chains account for approximately around 10% of a pectin (pectic substance). Most of the side chains are linked to position 4 of rhamnose and may be also linked to parts of galacturonic acids. In addition, as an example of a pectin structure, Fig. 1 shows an example of an apple pectin structure.

Examples of the pectin (pectic substance) of the present invention include protopectin, pectinic acid, pectic acid, and polygalacturonic acid. "Protopectin" is water-insoluble substance composed of cellulose, hemicellulose, lignin, protein, and the like linked to pectin in a plant tissue. The protopectin is solubilized by maturation (enzyme) to become pectinic acid. "Pectinic acid" is known as pectin in a narrow sense and is polygalacturonic acid of chain molecule (also referred to as polygalacturonan) composed of D-galacturonic acids polymerized by α-1,4 bonds and carboxyl groups that compose a part of the polygalacturonic acid and are partially methyl-esterified to be methoxyl groups. Hence, pectinic acid is a water-soluble substance with relatively low methoxyl group content. Furthermore, "pectic acid" is polygalacturonic acid composed of D-galacturonic acids polymerized by α-1,4 bonds. That is, this is pectinic acid containing no (or almost no) methoxyl groups.

Pectin is generally classified into high methoxyl pectin (also referred to as HM pectin) with an esterification degree of 50% or more and 100% or less, and low methoxyl pectin (also referred to as LM pectin) with an esterification degree of 0% or more and less than 50% according to the methyl-esterification ratio of carboxyl groups of galacturonic acids. However, types of pectin to be used in the inhibitor of the present invention are not limited by esterification degree. Any pectin such as high methoxyl pectin, low methoxyl pectin, or a mixture thereof can be used.

However, for use in the present invention, pectin with an esterification degree between 3% and 90% is preferable. High methoxyl pectin exists in a form resembling that of pectin existing in fruits, so that high methoxyl pectin with an esterification degree between 50% and 90% is more preferable, and high methoxyl pectin with an esterification degree between 65% and 85% is particularly preferable.

Furthermore, the molecular weight of pectin is also not particularly limited, but pectin with a low molecular weight is preferable in order to enhance solubility when it is added to a pharmaceutical composition, a cosmetic, or food and drink. For example, the origin of the pectin of the present invention is not particularly limited. The pectin may be derived from nature or it may be synthetic pectin. Particularly preferred pectins are derived from pectin-rich origins that are generally used as raw materials for pectin production, such as pericarps of citrus fruits, strained lees of apples, corn bran, wheat bran, sugar beet fibers, soybean fibers, and defatted rice bran.

Pectin production methods are not particularly limited. Generally, pectin production methods that are employed in industrial production can be implemented. For example, protopectins contained in strained lees of apples or pericarps of citrus fruits are hydrolyzed in a high-temperature acidic solution, followed by compression and filtration. The obtained filtrate is concentrated, pectin is precipitated with alcohol, and then washing, drying, and grinding can be conducted, thereby obtaining pectin in the form of powders. The thus obtained pectin is treated with acid, enzyme, or ammonia for demethylation to give low methoxyl pectin with a low esterification degree ("New Edition, Food Science and Technology Encyclopedia" ("*Shin-ban Shokuhin Kogyo So-go Jiten*" (edited by the Japanese Society for Food Science and Technology, KORIN Publishing Co., Ltd.))). In addition, for a laboratory-scale method of extracting pectin, "Pectic Substance, Protein·Nucleic acid·Enzyme" (Junichiro Ozawa, 15: pp. 888-894 (1970)) can be referred to.

Furthermore, pectin marketed as a food additive such as a gelatinizer, a stabilizer, or a thickener can also be directly utilized. Examples of high methoxyl pectin include UNIPECTINE type HM 1, UNIPECTINE type PG 109C, UNIPECTINE type SS 150, and UNIPECTINE type AYD 30 (all of which are produced by SKW BIOSYSTEMS) and GENU pectin type YM-150-LJ, GENU pectin type D Slow Set, and GENU pectin type BETA (all of which are produced by CP Kelco). Furthermore, examples of low methoxyl pectin include UNIPECTINE type OF 805, UNIPECTINE type AMP 285, UNIPECTINE type OB 700, and UNIPECTINE type AYD30 (all of which are produced by SKW BIOSYSTEMS), and GENU pectin type LM-12-1CG (produced by CP Kelco).

Moreover, the pectin obtained as described above may be a pectin wherein carboxyl groups form salts with various types of alkali such as sodium, calcium, and potassium.

In the present invention, a pectin hydrolysate can also be used as an active ingredient of the histamine release inhibitor. For example, a pectin hydrolysate can be obtained by hydrolysis of pectin using an enzyme such as 1% pectinase or a cell wall degrading enzyme or by hydrolysis of pectin by causing acid or alkali to act thereon. An example of 1% pectinase is polygalacturonase that can be caused to act at 37°C for 4 hours at pH 4.5. Furthermore, examples of a cell wall degrading enzyme that is preferably used in the present invention include Macerozyme (produced by YAKULT PHARMACEUTICAL IND CO., LTD) and Pectriase (produced by Kikkoman Corporation) that can be caused to act at 37°C for 4 hours at pH 5.5. As acid, for example, 1N to 2N hydrochloric acid or sulfuric acid can be used and caused to act at 100°C for 3 hours. Moreover, as alkali, for example, 0.05N sodium hydroxide can be used and caused to act at 0°C for 24 hours. However, these hydrolysis conditions do not limit the present invention.

The pectin hydrolysate of the present invention can be obtained by the above hydrolysis using an enzyme or acid or alkali. Examples of the hydrolysate include oligogalacturonic acid and/or polygalacturonic acid with various polymerization degrees. Furthermore, by purification through gel filtration, ultrafiltration, or the like, oligogalacturonic acid and/or polygalacturonic acid with a desired polymerization degree can be obtained. Of these, oligogalacturonic acid and/or polygalacturonic acid with a polymerization degree ranging from 2 to 100 is preferable and in particular, oligogalacturonic acid with a polymerization degree ranging from 2 to 10 is preferable. In addition, such oligogalacturonic acid and/or polygalacturonic acid may be in a state wherein the side chain portion composed of neutral sugars such as arabinose, xylose, fucose, rhamnose, and galactose and portions of the side chain portion are bound depending on the site and the strength of hydrolysis by an enzyme or acid or alkali.

Furthermore, an oligosaccharide comprising 1 type or 2 or more types selected from the group consisting of galacturonic acid, rhamnose, arabinose, xylose, fucose, and galactose as a constituent can be obtained by sufficiently carrying out hydrolysis using an enzyme or acid or alkali. Such oligosaccharides are also included in the hydrolysate of the present invention. Of these, in particular, an oligosaccharide of arabinose is particularly preferred.

In addition, commercially available products of these oligosaccharides, the above oligogalacturonic acid, and the above polygalacturonic acid can also be utilized. For example, an oligosaccharide of arabinose is marketed by Megazyme International Ireland, Ltd.

Whether or not pectin has action to lower histamine concentration in blood can be confirmed by, for example, letting subjects experience intake of the pectin for 3 weeks, collecting blood before the start of intake, at the end of intake, and 2 weeks after the end of intake, and measuring histamine concentration in blood. By this method, we have confirmed that histamine concentration in blood is significantly lowered by the intake of pectin.

As described above, a pectin or a salt thereof or a pectin hydrolysate has a feature of lowering histamine concentration in blood. Hence, the histamine release inhibitor comprising a pectin or a salt thereof or a pectin hydrolysate as an active ingredient can be directly administered when it is used for preventing and/or treating diseases such as allergic rhinitis, atopic dermatitis, urticaria, bronchial asthma, peptic ulcer, and pollinosis.

Furthermore, the histamine release inhibitor comprising a pectin or a salt thereof or a pectin hydrolysate as an active ingredient can be mixed with pharmaceutically acceptable various carriers and other ingredients to produce a pharmaceutical composition for preventing and/or treating diseases such as allergic rhinitis, atopic dermatitis, urticaria, bronchial asthma, peptic ulcer, and pollinosis by a known method.

Examples of the dosage form of a histamine-release-inhibiting pharmaceutical composition include oral agents such as tablets, powders, granules, capsules, and syrups, parenteral agents such as injections, and external preparations such as ointment, cream, lotion, and gel. Formulation can be carried out by a standard method. Regarding the form of administration, the composition can be administered to animals including humans, orally, parenterally (e.g., intravenous, intra-arterial, intramuscular, intraperitoneal, or subcutaneous administration), via rectum, nasal cavity, eyes, or the like, or by application to skin. The form of administration is not particularly limited. However, there may be a case where long-term administration is required in addition to preventive administration. When these cases are taken into consideration, oral administration or application to skin is preferable because of convenience in administration and low mental and physical burdens to patients.

As long as there are no problems in simultaneous administration, other known antiinflammatory agents and other known allergy inhibitors can be used in combination as other ingredients.

As a pharmaceutically acceptable carrier to be used for the pharmaceutical composition of the present invention, carriers that are generally used as materials for preparations, such as excipients, extending agents, binders, moistening agents, disintegrating agents, surfactants, lubricants, dispersing agents, buffers, preservatives, or the like are appropriately combined and prescribed, so as to allow production of the pharmaceutical composition. If necessary, additives such as antiseptics, antioxidants, colorants, corrigents, aromatics, and coatings can also be used.

Specifically, as examples of a solid carrier, starch, lactose, carboxymethylcellulose, corn starch, light anhydrous silicic acid, crystalline cellulose, dextrin, hydroxypropylcellulose, polyvinylpyrrolidone, magnesium stearate, calcium stearate, or talc can be used. Furthermore, as examples of a liquid carrier, distilled water, saline, glucose aqueous solution, ethanol, propylene glycol, or the like, sesame oil, or corn oil can be used. These carriers may be appropriately selected depending on the dosage form or the form of administration.

The dose of the pectin or the salt thereof or the pectin hydrolysate of the present invention may be any dose, as long as it can inhibit the release of histamine. Such dose can be administered once or at several separate instances; that is, once to several instances per day. This is preferably determined appropriately depending on purposes for use (treatment and/or prevention), age and weight of subjects, administration methods, and the like.

Furthermore, by adding the histamine release inhibitor comprising a pectin or a salt thereof or a pectin hydrolysate as an active ingredient to a cosmetic as an ingredient, a cosmetic for preventing diseases such as allergic rhinitis, atopic dermatitis, urticaria, bronchial asthma, peptic ulcer, and pollinosis, or alleviating the symptoms of these diseases can be obtained. In the present invention, examples of a cosmetic include toilet water, emulsions, cream, and bath agents, but are not limited thereto. In these cosmetics, if necessary, raw materials that are generally used for cosmetics, such as oil, moisturizing agents, ultraviolet absorption agents, antioxidants, and preservatives can be appropriately compounded therein. Moreover, other anti-inflammatory raw materials that are generally used for cosmetics, such as glycyrrhizic acid and diphenhydramine hydrochloride, can also be added if necessary.

Furthermore, by adding the histamine release inhibitor of the present invention to food and drink as a raw material, food and drink having activity of inhibiting histamine release can be provided. Examples of such food and drink include: beverages such as refreshing drinks, carbonated drinks, lactic acid beverages, juice, and nutritional drinks; jam, jelly, cream, ice cream, and sweet stuff and ice such as candies, buiscuits, pudding, crunch chocolate, and cornflakes; dairy products such as yogurt; and seasonings such as mayonnaise and dressing, but they are not limited thereto. The compounding ratio of the histamine release inhibitor of the present invention in food and drink is not particularly limited, and can be appropriately determined according to the physical properties and the like of food and drink to which the inhibitor is added. The food and drink of the present invention contain large amounts of the histamine release inhibitor, which is impossible to obtain by direct intake of fruits alone, so that the food and drink are very effective for preventing diseases such as allergic rhinitis, atopic dermatitis, urticaria, bronchial asthma, peptic ulcer, and pollinosis, and alleviating the symptoms of these diseases.

No upper limits are particularly determined for the dose, the amount used, or the intake of the above pharmaceutical composition, cosmetic, or food and drink, because pectin is classified as belonging to a class of food additives for which it is not required by FAO/WHO to determine allowable intake per day. According to the forms for use, purposes, conditions of patients, and the like, the dose (intake) can be appropriately determined. For example, in the case of oral administration or intake, a dose (intake) between 0.1 g and 50 g per day, preferably a dose (intake) between 6 g and 36 g per day, and more preferably a dose (intake) between 8 g and 20 g per day can be employed.

In the case of pharmaceutical compositions for parenteral administration and cosmetics, the content of the histamine release inhibitor of the present invention can be determined to range from 0.0001% to 50.0% (w/w) in a preparation, is preferably 0.001% or more, and is further preferably 0.01% or more, and is even further preferably 0.1% or more. The upper limit is determined depending on the physical and chemical properties of preparations. In addition, when the inhibitor is contained in a bath agent, the agent may be formulated to achieve the above concentration range at the time of use.

No considerable side effects have been reported concerning the histamine release inhibitor of the present invention at this time. Hence, cosmetics and food and drink comprising the histamine release inhibitor can be continuously used for or taken by animals including humans for the purpose of preventing and/or treating diseases such as allergic rhinitis, atopic dermatitis, urticaria, bronchial asthma, peptic ulcer, and pollinosis.

Examples will be shown below to explain the present invention in detail, but are not intended to limit the present invention.

### [Example 1] Confirmation of changes in blood histamine concentration due to pectin intake

Subjects were asked to take pectin for 3 weeks. Blood was collected before the start of intake, at the end of intake, and 2 weeks after the end of intake, and histamine concentration in blood was measured for determination.

The pectin used herein was granular pectin that was appropriate for intake and had been prepared based on the method for producing a thickening polysaccharide material as disclosed in the JP Patent Publication (Kokai) 2000-014336 A. Specifically, an apple-derived high methoxyl pectin powder product, Apple Pectin HM-1 (with a pectin content of 90.5% and an esterification degree between 72 and 76) (produced by SKW BIOSYSTEMS, France) and anhydrous crystal glucose were compounded at a 1:1 ratio to produce mixed powders, and then the powders were directly heated under anhydrous conditions. The thus obtained pectin granules were used for this experiment.

Subjects (14 adults: 47 years old on average, 25 to 68 years old, 11 males and 3 females) were asked to take 22.2 g of pectin granules (equivalent to 9.99 g of pectin) per day as a standard for a period of 3 weeks. Blood was collected before the start of intake, at the end of intake, and 2 weeks after the end of intake, and plasma histamine concentration was measured. In addition, the experiment was conducted for a period of 7 continuous weeks, including the 2 weeks before the start of intake. During the experimental period, the intake of fruits was limited for the purpose of limiting the intake of pectin as a dietary limitation. In addition, the presence or the absence of drug therapy was also investigated.

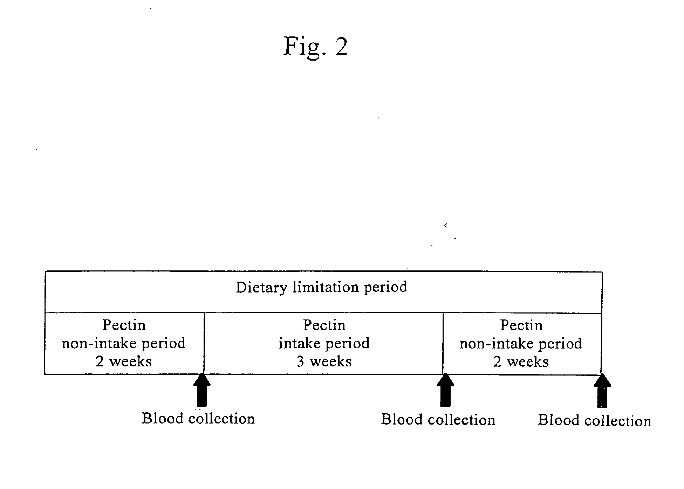

The experiment design including the periods of pectin intake and blood collection is shown in Fig. 2.

The quantitative measurement method of histamine concentration in plasma was carried out as described below. 2 ml of blood was collected using an EDTA2K blood collection tube. Within 20 minutes following blood collection, the collected blood was centrifuged (4°C, 1500 rpm, 15 minutes). 0.5 ml of plasma was dispensed, the resultant was immediately frozen, and it was then used for quantitative measurement of histamine. Histamine was quantitatively measured using a histamine ELISA kit (Immunotech). The average value of histamine concentration for each period is shown in Table 1. A significant difference between the results obtained after 3 weeks of intake of pectin granules and those obtained before the start of intake or those obtained 2 weeks after the end of intake was found through the t-test.

**Table 1**

| | Histamine concentration (ng/ml) | t-test |
|---|---|---|
| Before intake (pectin non-intake) | 0.70 | P<0.01 |
| Pectin intake | 0.53 | P<0.05 |
| After intake (pectin non-intake) | 0.67 | |

As is clear from the results in Table 1, compared with the result obtained before the start of the intake of pectin granules, the average value of histamine concentration in plasma measured after the intake significantly decreased from 0.70 ng/ml to 0.53 ng/ml (P<0.01). Furthermore, at 2 weeks after the end of intake, the average value of histamine concentration in plasma had significantly increased to 0.67 ng/ml, which was close to the concentration measured before the intake (P<0.05).

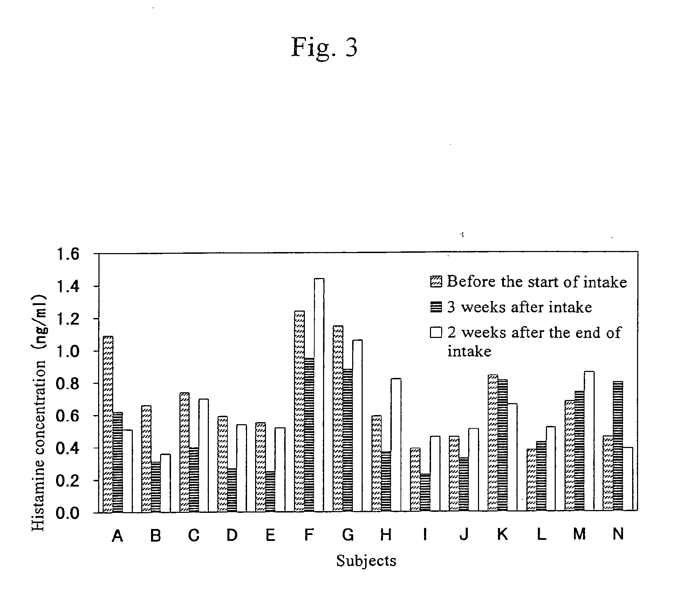

Furthermore, fluctuations in the blood histamine concentrations of each of all subjects are shown in Fig. 3. 11 out of 14 subjects showed decreases in histamine concentration due to the intake of pectin granules.

The above results revealed that the intake of pectin lowers histamine concentration in blood.

### [Example 2] Preparation of pharmaceutical composition

With the following composition, tablets were produced by direct compression molding.

| | |
|---|---|
| High methoxyl pectin powder | 100 mg |
| Crystalline cellulose | 400 mg |
| Lactose | 90 mg |
| Hydroxypropylcellulose-L | 4 mg |
| Magnesium stearate | 3 mg |
| Total | 500 mg |

### [Example 3] Preparation of cream

With the following compounding ratio, cream (cosmetic) was produced according to a standard method.

### (Compounding ratio)

| | |
|---|---|
| High methoxyl pectin powder | 0.5% |
| Stearic acid | 8.0% |
| Stearyl alcohol | 4.0% |
| Butyl stearate | 6.0% |
| Propylene glycol | 5.0% |
| Glyceryl monostearate | 2.0% |
| Potassium hydroxide | 0.4% |
| Antiseptic | Optimum dose |
| Antioxidant | Optimum dose |
| Aromatic | Optimum dose |

Purified water was added to the above composition to 100%.

### [Example 4] Preparation of food and drink

Candy with the following composition was produced.

| | |
|---|---|
| High methoxyl pectin powder | 0.5% |
| Sugar | 47.5% |
| Starch syrup | 49.0% |
| Aromatic | 1.0% |
| Water | 2.0% |

### Industrial Applicability

The histamine release inhibitor of the present invention has the effect of lowering histamine concentration in blood, useful for treating and/or preventing allergic rhinitis, atopic dermatitis, urticaria, bronchial asthma, peptic ulcer, pollinosis, and the like, has high safety, and can be continuously used for a long period.

All publications, patents, and patent applications cited herein are incorporated herein by reference in their entirety.

## Claims

1. A histamine release inhibitor, comprising a pectin or a salt thereof or a pectin hydrolysate as an active ingredient.

2. The histamine release inhibitor of claim 1, wherein the pectin is a high methoxyl pectin having an esterification degree of 50% or more and 90% or less.

3. The histamine release inhibitor of claim 1, wherein the pectin is a low methoxyl pectin having an esterification degree of 3% or more and less than 50%.

4. The histamine release inhibitor of claim 1, wherein the pectin hydrolysate is oligogalacturonic acid and/or polygalacturonic acid with a polymerization degree between 2 and 100.

5. The histamine release inhibitor of claim 1, wherein the pectin hydrolysate is an oligosaccharide comprising 1 type or 2 or more types selected from the group consisting of galacturonic acid, rhamnose, arabinose, xylose, fucose, and galactose as a constituent.

6. The histamine release inhibitor of any one of claims 1 to 5, which is for preventing and/or treating allergic rhinitis, atopic dermatitis, urticaria, bronchial asthma, peptic ulcer, and/or pollinosis.

7. A pharmaceutical composition, comprising the inhibitor of any one of claims 1 to 6.

8. A cosmetic, comprising the inhibitor of any one of claims 1 to 6.

9. Food and drink, comprising the inhibitor of any one of claims 1 to 6.
